# NOUVEAU FASCICULE DE BREVET EUROPEEN

(11) **EP 0 605 381 B2**
(45) Date de publication et mention de la décision concernant l'opposition: **23.10.2002**
(45) Mention de la délivrance du brevet: 21.01.1998
(21) Numéro de dépôt: 93870238.8
(22) Date de dépôt: 21.12.1993
(51) Int. Cl.: C08L 53/02, A61L 29/00

(54) **Compositions de polymères pour applications médicales**
Polymerzusammensetzung zur medizinischen Anwendungen
Composition of polymers for medical applications

(30) Priorité: 30.12.1992 BE 9201171
(43) Date de publication de la demande: 06.07.1994
(73) Titulaire: ATOFINA Research, 7181 Seneffe (Feluy) (BE)
(72) Inventeur: Lecouvet, Nicolas, B-1420 Braine-l'Alleud (BE); Simon, Serge, B-1410 Waterloo (BE)

(56) Documents cités:
- EP-A- 0 189 911
- EP-A- 0 368 141
- DE-A- 1 934 348
- FR-A- 2 323 737
- JP-A- 5 620 515
- US-A- 3 823 203
- US-A- 4 048 254

## Description

La présente invention concerne une composition de polymères appropriée en particulier aux applications médicales. En particulier, la présente invention concerne une composition de polymères qui peut avantageusement remplacer le chlorure de polyvinyle (PVC) dans de nombreuses applications médicales.

L'utilisation du PVC dans les applications médicales est bien connue. On peut citer, entre autres, les pochettes de sang, les pochettes de solutions intra-veineuse, les tuyaux.

Les propriétés requises pour ce type de matériaux sont les suivantes :
- Non-perméabilité aux gaz et liquides afin d'éviter tout passage de microorganismes au travers du matériau.
- Porosité permettant la stérilisation au moyen d'oxyde d'éthylène ou par passage dans un autoclave.
- Stabilité du matériau après stérilisation par irradiations.
- Résistance, en particulier bonnes flexibilité et résilience.
- Processabilité.
- Transparence.
- Sécurité de manière générale, à savoir non toxicité, non carcinogénicité et surtout ne pas être nuisible à l'environnement.

La plupart des matériaux polymères utilisés dans les applications médicales sont à usage unique. Dès lors, il est très important de prévoir une destruction "propre" de ces matériaux. Cela n'est pas le cas avec le PVC qui conduit à la formation de sous-produits très corrosifs et toxiques lors de son incinération.

Il existe dès lors un besoin dans le domaine médical pour un nouveau matériau répondant aux exigences requises en matière de propriétés physico-chimiques telles qu'énumérées ci-avant et, en plus, "propre" à l'égard de l'environnement.

La demanderesse a maintenant trouvé une nouvelle composition de polymères qui répond à toutes ces exigences.

La composition de polymères conformément à la présente invention comprend un mélange de copolymère bloc styrène-butadiène (S-B) et de copolymère bloc styrène-isoprène (S-I). En particulier, la composition de polymères conformément à la présente invention comprend un mélange de copolymère bloc styrène-butadiène (S-B) dont la teneur en butadiène est comprise entre 20 et 80% en poids, de préférence entre 40 et 70% en poids, et de copolymère bloc styrène-isoprène (S-I) dont la teneur en isoprène est comprise entre 20 et 95% en poids, de préférence entre 60 et 90% en poids.

Les copolymères styrène-butadiène et styrène-isoprène peuvent chacun être choisis parmi les copolymères dibloc (SB, SI) ou les copolymères tribloc (SBS, SIS). Toutefois on utilisera de préférence les copolymères tribloc styrène-butadiène et styrène-isoprène (SBS, SIS).

Les copolymères blocs utilisés dans la présente invention peuvent être linéaires, radiaux ou multibranchés selon que l'on utilise ou non un agent de couplage di- ou plurifonctionnel; il est évident que l'on peut également utiliser dans le procédé de l'invention des copolymères blocs linéaires préparés sans agent de couplage. Selon un mode d'exécution préféré de l'invention, on utilise comme composant du mélange un copolymère bloc radial de type styrène-butadiène et un copolymère bloc linéaire de type styrène-isoprène.

En particulier, la composition selon la présente invention comprend un mélange de copolymères styrène-butadiène : styrène-isoprèns dont les proportions en poids varient de 60:40 à 20:80. Selon un mode préféré de la présente invention, les proportions en poids de copolymères styrène-butadiène : styrène-isoprène sont comprises entre 50:50 et 30:70.

Le brevet DE 1934348 divulgue des mélanges analogues pour des applications de cordonnerie. La présente invention adapte ce type de mélange à des applications médicales.

La Demanderesse a trouvé de manière tout-à-fait inattendue que les matériaux répondant à la composition du mélange de polymères de la présente invention possèdent de très bonnes propriétés physiques. De plus, la Demanderesse a constaté qu'après irradiation des dits matériaux, ceux ci présentent encore de bonnes propriétés physiques et même dans certains cas des propriétés améliorées. Ceci représente un avantage considérable par rapport aux matériaux tels que le PVC qui, après irradiation, présentent des propriétés physiques très instables.

La Demanderesse a également constaté que la processabilité des mélanges de polymères suivant la présente invention s'effectuait de manière très aisée. Toutefois pour certaines applications médicales, il peut être souhaitable d'ajouter au mélange des additifs anti-collants afin d'améliorer encore la processabilité. Parmi ces additifs, on peut citer le monostéarate d'éthylène glycol, l'éthylène disstéaramide et l'érucamide. Ces additifs peuvent être ajoutés aux mélanges de la présente invention en des quantités pouvant aller jusqu'à 1000 ppm. De préférence ces quantités seront comprises entre 250 et 750 ppm.

Les compositions de polymères selon la présente invention sont particulièrement appropriées à être utilisées dans les applications médicales comme définies dans la présente demande de brevet. En particulier, ces compositions sont utilisées pour fabriquer des tuyaux à usage externe, par exemple, les tuyaux permettant le transport de solutés et de sang ainsi que les tuyaux de drainage.

Le mélange des composants polymériques peut être effectué par n'importe quelle méthode conventionnelle. Habituellement, on introduit dans un mélangeur rotatif les composants sous forme de granules et on fait passer ce mélange chauffé à l'état plastique dans une extrudeuse-granulateur.

La composition de polymère obtenue est ainsi prête à être utilisée pour des procédés de transformation tels que l'extrusion, le thermoformage de feuilles extrudées et le moulage sous-pression.

Les exemples suivants servent à illustrer la présente invention.

### Exemples

### a) Composition du mélange

- Copolymère tribloc styrène-butadiène (SBS) dont les propriétés sont données ci-dessous (mesurées à partir d'un specimen injecté) :
   - structure radiale
   - teneur en styrène : 40% en poids
   - résistance à la traction : 20 MPa (norme ASTM D 412)
   - index d'écoulement (200°C - 5 kg) : 6 g/10 min (norme ASTM D 1238)
   - résistance à la déchirure : 11 N/mm (norme ASTM D 624)
   - point de ramolissement VICAT (50°C -1 kg) : 53°C (norme ASTM D 1525)
   - densité (23°C) : 0.96 g/cm³ (norme ASTM D 0792)
   - dureté shore D : 38 (norme ASTM D 2240)
- Copolymère tribloc styrène-isoprène (SIS) dont les propriétés sont données ci-dessous (mesurées à partir d'un specimen injecté) :
   - structure linéaire
   - teneur en styrène : 15% poids
   - index d'écoulement (190°C - 5 kg) : 7 g/10 min

### b) Conditions opératoires

Les deux composants (sous forme de granules) sont mélangés dans un malaxeur rotatif lent à une vitesse de 100 t/min.
Ensuite la matière est introduite dans une extrudeuse-film avec les conditions opératoires suivantes :
pression (bars): 110
température (°C) : 185
vitesse de la vis (t/min) : 105
épaisseur de la filière (µm) : 500
largeur de la filière (cm) : 9

### c) Propriétés du matériau

Les caractéristiques du matériau polymère sont indiquées dans le tableau 1.

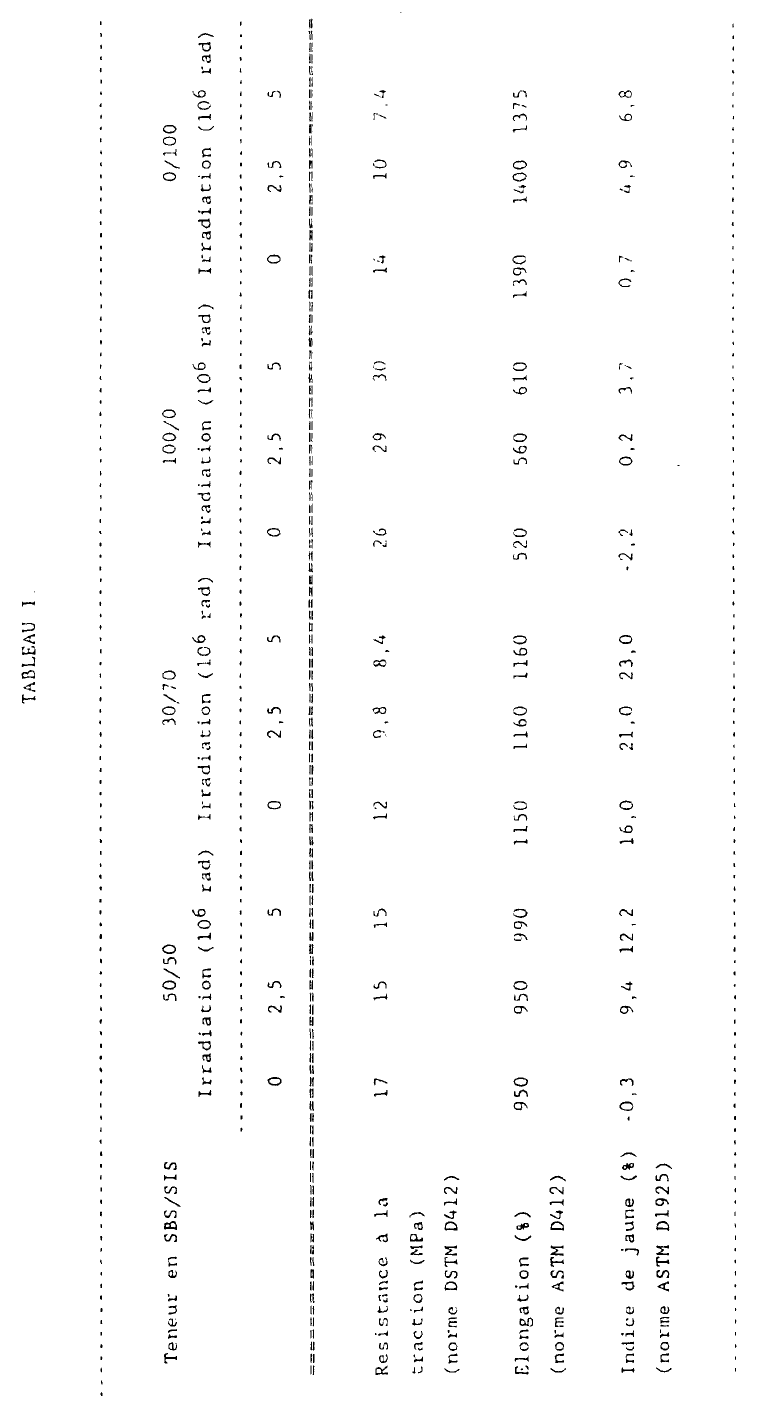

## Revendications

1. Utilisation d'une composition de polymères dans des applications médicales choisies parmi les pochettes de sang, les pochettes de solutions intra-veineuses et les tuyaux **caractérisée en ce que** cette composition comprend un mélange de copolymère bloc styrène-butadiène et de copolymère bloc styrène-isoprène.

2. Utilisation selon la revendication 1 **caractérisée en ce que** le copolymère styrène-butadiène a une teneur en butadiène comprise entre 20 et 80% en poids, de préférence entre 40 et 70% en poids, et le copolymère styrène-isoprène a une teneur en isoprène comprise entre 20 et 95% en poids, de préférence entre 60 et 90% en poids.

3. Utilisation selon l'une quelconque des revendications 1 à 2 **caractérisée en ce que** le copolymère styrène-butadiène et le copolymère styrène-isoprène sont des copolymères triblocs.

4. Utilisation selon l'une quelconque des revendications 1 à 3 **caractérisée en ce que** le copolymère styrène-butadiène est un copolymère à structure radiale.

5. Utilisation selon l'une quelconque des revendications 1 à 4 **caractérisée en ce que** le copolymére styrène-isoprène est un copolymère à structure linéaire.

6. Utilisation selon l'une quelconque des revendications 1 à 5 **caractérisée en ce que** les proportions en poids de copolymères styrène-butadiène : styrène-isoprène sont comprises entre 60:40 et 20:80.

7. Utilisation selon la revendication 6 **caractérisée en ce que** les proportions en poids de copolymères styrène-butadiène : styrène-isoprène sont comprises entre 50:50 et 30:70.

8. Utilisation selon l'une quelconque des revendications 1 à 7 **caractérisée en ce qu'**on ajoute au mélange de copolymères un ou plusieurs additifs anti-collants qui peuvent être choisis parmi le monostéarate d'éthylène glycol, l'éthylène disstéaramide et l'érucamide.

## Patentansprüche

1. Verwendung einer Polymerzusammensetzung für medizinische Anwendungen ausgewählt aus den Blutbeuteln, Beuteln für intravenöse Lösungen und Schläuchen, **dadurch gekennzeichnet, dass** diese Zusammensetzung ein Gemisch aus Styrol-Butadien Blockcopolymers und eines Styrol-Isopren Blockcopolymers enthält.

2. Verwendung nach Anspruch 1, **dadurch gekennzeichnet, daß** das Styrol-Butadien Copolymer einen Gehalt an Butadien zwischen 20 und 80 Gew.-%, vorzugsweise zwischen 40 und 70 Gew.-% aufweist und daß das Styrol-Isopren Copolymer einen Gehalt an Isopren zwischen 20 und 95 Gew.-%, vorzugsweise zwischen 60 und 90 Gew.-% aufweist.

3. Verwendung nach einem der Ansprüche 1 bis 2, **dadurch gekennzeichnet, daß** das Styrol-Butadien Copolymer und das Styrol-Isopren Copolymer Triblockcopolymere sind.

4. Verwendung nach einem der Ansprüche 1 bis 3, **dadurch gekennzeichnet, daß** das Styrol-Butadien Copolymer ein Copolymer mit einer strahlenförmigen Struktur ist.

5. Verwendung nach einem der Ansprüche 1 bis 4, **dadurch gekennzeichnet, daß** das Styrol-Isopren Copolymer ein Copolymer mit einer linearen Struktur ist.

6. Verwendung nach einem der Ansprüche 1 bis 5, **dadurch gekennzeichnet, daß** die Gewichtsverhältnisse der Styrol-Butadien : Styrol-Isopren Copolymere zwischen 60 : 40 und 20 : 80 liegen.

7. Verwendung nach Anspruch 6, **dadurch gekennzeichnet, daß** die Gewichtsverhältnisse der Styrol-Butadien : Styrol-Isopren Copolymere zwischen 50 : 50 und 30 : 70 liegen.

8. Verwendung nach einem der Ansprüche 1 bis 7, **dadurch gekennzeichnet, daß** dem Gemisch der Copolymere eines oder mehrere Anti-Haftadditive zugesetzt werden, die aus Ethylenglykolmonostearat, Ethylendistearamid und Erucaamid ausgewählt werden.

## Claims

1. Use of a polymer composition in medical applications chosen among bags for blood, bags for intravenous solutions and tubes, **characterized in that** this composition comprises a mixture of styrene-butadiene block copolymer and styrene-isoprene block copolymer.

2. Use according to Claim 1, **characterised in that** the styrene-butadiene copolymer has a butadiene content of between 20% and 80% by weight, preferably between 40% and 70% by weight, and the styrene-isoprene copolymer has an isoprene content of between 20% and 95% by weight, preferably between 60% and 90% by weight.

3. Use according to either of Claims 1 to 2, **characterised in that** the styrene-butadien copolymer and the styrene-isoprene copolymer are triple-block copolymers.

4. Use according to any one of Claims 1 to 3,
**characterised in that** the styrene-butadiene copolymer is a copolymer with a radial structure.

5. Use according to any one of Claims 1 to 4,
**characterised in that** the styrene-isoprene copolymer is a copolymer with a linear structure.

6. Use according to any one of Claims 1 to 5,
**characterised in that** the weight ratios of the styrene-butadiene copolymers to the styrene-isoprene copolymers are between 60:40 and 20:80.

7. Use according to any one of Claim 6, **characterised in that** the weight ratios of the styrene-butadiene copolymers to the styrene-isoprene copolymers are between 50:50 and 30:70.

8. Use according to any one of Claims 1 to 7,
**characterised in that** one or more anti-adhesive additives are added to the mixture, which may be selected from ethylene glycol monostearate, ethylene disstearamide and erucamide.
